# EUROPEAN PATENT APPLICATION

(11) **EP 1 387 292 A1**
(43) Date of publication of application: **04.02.2004**
(21) Application number: 02016796.1
(22) Date of filing: 26.07.2002
(51) Int. Cl.: G06F 17/30, G06F 19/00

(54) **Method and apparatus for combining data of biological sequences into a non-redundant data source**

(71) Applicant: LION bioscience AG, 69123 Heidelberg (DE)
(72) Inventor: Ohr, Christian, 69198 Schriesheim-Altenbachh (DE)
(74) Representative: Schohe, Stefan

(57) **Abstract**

The invention provides a method for establishing or modifying a data source comprising a plurality of entries related to biological sequences that are non-redundant with regard to said sequences on the basis of a plurality of data sets of one or more basic data sources, each of said data sets comprising a biological sequence, said method comprising the steps of:
- retrieving for one or more data sets a biological sequence contained in the data set and generating a hash key from the biological sequence thus retrieved by applying a collision-free hash function, said hash function mapping the data representing said sequence onto a message of a length shorter than the length of the original data representing the sequence,
- for each of said data sets, adding information for retrieving information from said data set to an entry in a reference data source uniquely related to the hash key generated from said sequence contained in said data set, wherein a new entry in said reference data source is provided which comprises one unique hash key and information for retrieving the data set or data sets comprising the sequence from which said hash key was generated, if said reference data source does not comprise an entry related to said hash key,
such that each entry in said reference data source is uniquely identified by a hash key generated from a sequence.

The invention also relates to a corresponding computer system and a method of updating a non-redundant data source using a reference data source.

## Description

The invention relates to non-redundant databases derived from redundant information comprised in one or more basic data sources, especially in the field of bioinformatics.

A redundancy in the available information may arise, because the basic data source itself comprises redundant information or because there is a redundancy between information contained in the various data sources. The problem of redundancy is especially relevant in bioinformatics, where one goal is to be able to retrieve through one single interface information derived from a variety of data sources, reaching from big databases such as GENBANK or SWISSPROT down to small databases maintained by local research groups. One technique for achieving this goal is to create a unified database comprising links to the underlying basic data sources. It is, of course, desirable that this unified database is non-redundant as to its entries. Since various data sources may relate to the same entity by different names or in a different context, it is usually hard to determine in an automated procedure whether two entries relate to the same thing.

When the object of interest are biological sequences, such as nucleotide sequences or protein sequences, there is one data item that is unique for all related entries, namely the sequence itself. In principle, one can determine whether two entries, be it within the same data source, be it in different data sources, relate to the same sequence just by comparing the sequence. Based on this idea, a prior art algorithm was established that essentially runs as follows:
- input sequence information from a variety of databases,
- identify entries in said databases which relate to the same sequence and combining them into one cluster, identified by a unique identifier and
- creating a non-redundant database from entries related to said clusters.

An application that is working according to this algorithm is the nrdb application provided by the National Center for Biotechnology Information (NCBI), National Library of Medicine, Building 38A Bethesda, MD 20894, USA. Further information about the nrdb tool may be found at http://www.ncbi.nlm.nih.gov and ftp://ftp.ncbi.nih.gov/pub/nrdb/README.

With today's database sizes the nrdb application has a high demand on system memory (typically in the 10 GB range for handling several millions of sequences) and execution time (typically in the range of about 10 hours with major current databases in biotechnology). Since databases in biotechnology show exponential growth, performance of the nrdb algorithm is strongly degraded. Another shortcoming of the nrdb application is that the non-redundant database created by this algorithm can only be updated by recomputing the entire database. Frequently, big databases, such as GENBANK or SWISSPROT are updated only in intervals of several months. New information arising in the meantime is contained in supplementary databases, as long as the entire database has not been updated. It would be extremely desirable to be able to update a non-redundant database related to sequences on the basis of these supplementary databases. This is, however, something the nrdb algorithm does not allow for.

In the field of cryptography, so-called one-way hash functions are known. These hash functions are designed in a way that it is virtually impossible to invert the function. "Virtually impossible" means that, considering the computer resources presently available, any attempt to invert the function by means of a computer has a negligible probability of success. There are also known so-called collision-free one-way hash functions in cryptography where it is virtually impossible (in the sense explained above) to find two elements of the mathematical set that is mapped by the function that result in the same value of the function. Such one-way hash functions are used to provide a so-called digital fingerprint of a text. If e.g. a text is stored or forwarded in electronic form and retrieved in character form later on, it is possible to verify whether this text was altered while being stored or being sent, if the value of the hash function, when applied to the original text, is known. One simply applies the hash function to the text as retrieved. If this value is identical to the original value of the hash function, one can be reasonably certain that the text was not changed, especially if the hash function is collision-free.

It is the object of the present invention to establish and maintain a non-redundant database with less memory consumption and more algorithmic efficiency, which ideally would also allow for incremental updates.

According to the invention, this object is accomplished by a method for establishing or modifying a data source comprising a plurality of entries related to biological sequences that are non-redundant with regard to said sequences, on the basis of a plurality of data sets of one or more basic data sources, each of said data sets comprising a biological sequence, said method comprising the steps of:
- retrieving for one or more data sets a biological sequence contained in the data set and generating a hash key from the biological sequence thus retrieved by applying a collision-free hash function, said hash function mapping the data representing said sequence onto a message of a length shorter than the length of the original data representing the sequence,
- for each of said data sets, adding information for retrieving information, especially the sequence, from said data set to an entry in a reference data source uniquely related to the hash key generated from said sequence contained in said data set, wherein a new entry in said reference data source is provided which comprises one unique hash key and information for retrieving the data set or data sets comprising the sequence from which said hash key was generated, if said reference data source does not comprise an entry related to said hash key prior to said adding step,
such that each entry in said reference data source is uniquely identified by a hash key generated from a sequence.

One will note that the reference data source is already non-redundant with regard to sequences. One may therefore keep this reference data source as a non-redundant data source for sequences, given the case after adding the sequences themselves. One may, however, also use the reference data source only for reference purposes and create a non-redundant data source comprising said sequences and not comprising said hash keys on the basis of said reference data source. In the latter case, the method according to the invention may comprise the step of establishing a non-redundant data source or modifying an existing non-redundant data source on the basis of the reference data source established or modified by the steps mentioned previously.

Unless a new reference data source is created, the method will usually comprise the step of determining whether said reference data source comprises an entry related to said hash key generated from said sequence. If a new reference data source is created, e.g. if the reference data source is generated for the first time from the basic data sources, one will in a first step create an entry related to the first hash key in the manner indicated above and in all subsequent steps compare whether subsequent entries correspond to this entry (or other entries created in the meantime) and only create new entries, if there is not yet an entry related to the same hash key.

In the context of this invention, a data set is to be understood as a group of data structurally related to each other in a manner that said group of data can be accessed independently of other data outside said group. A data set in this sense may, for example, be an entry in a database or a part thereof that can be accessed individually, or even the data content of a file that can be individually accessed, such as e.g. an HTML file. A data source in the sense of the invention is to be considered as any entity comprising one or more data sets, e.g. a database, a flat file comprising a plurality of entries or even a file comprising a data set, which file may or may not be a part of a larger directory.

In many biological applications the basic data sources are databases in a flat file format, especially in the FASTA format. Details of the FASTA format can be found at http://www.molbio.ox.ac.uk/help/formatexamples#fasta

The sequence in said basic data sources is represented by a succession of data units representing the elements of the sequence, especially a succession of data units representing a lexical sequence, wherein the elements of the sequence are designated by letters, such as the letters A, G, T and C for nucleotide sequences.

In the context of this invention, a hash function is to be understood as a function that maps a message onto a message of shorter length. A collision-free hash function is to be understood as a hash function for which the probability that two different sequences are mapped on the same value is virtually zero, i.e. the function effectively uniquely assigns a unique hash key to each sequence. The hash function according to this invention may especially be a one-way function. Since, as far as data processing is concerned, sequences just correspond to a random succession of characters and especially new sequences have to be accommodated, the collision-free hash function has to perform this task irrespective of the content of the sequence, i.e. any sequence occurring, be it known or not, will be mapped on a hash key that is effectively unique for this sequence.

Functions that meet these requirements are well-known in the field of cryptography, though for different purposes. A hash function used in cryptography will work irrespective of the form or content of the data it is applied on, i.e. if such a hash function is applied to two arbitrary successions of characters, the likelihood of the case that the value of the hash function will be the same is virtually zero. For further details regarding cryptographic hash functions reference is made to the standard textbooks in cryptography, see also Menezes, van Oorschot, Vanstone: Handbook of Applied Cryptography CRC Press, ISBN: 0-8493-8523-7, http://www.cacr.math.uwaterloo.ca/hac/. In the field of cryptography, the terms one-way function and collision-free are defined in that it is "computationally infeasible" to invert the function or to find two arguments of the function resulting in the same value, respectively. Due to the different purpose these functions serve in cryptography, these definitions are slightly different from the definition used here, since the possibility of inverting the hash function is not an issue in the present invention.

However, if a cryptographic hash function is collision-free in the sense of cryptography, it is usually also a collision-free hash-function in the sense of this invention, since the probability that two arguments of the function will yield the same value is virtually zero if it is computationally infeasible to determine such two values. It should, however, be noted that a hash function in the sense of this invention does not necessarily have to be a hash function in the sense of cryptography. For example, if the hash function is no longer collision-free in the sense of cryptography, because the capacity of the available computer resources has increased, it may still be collision-free in the sense of the invention.

The advantage of using a hash function in the sense of the invention is that a sequence is assigned a unique hash key (also called digital fingerprint) irrespective of whether the sequence was known before and/or whether said sequence occurred prior to other sequences. An important feature of a hash function according to the invention is that the value of the hash function is independent of the order in which the sequences appear. This is to be contrasted to index listing in the order of appearance, wherein an sequence receives an index that is incremented by 1 over the index of the last sequence that occurred previously. With this kind of indexing, the index of a sequence does not depend only on the sequence, but also on the order in which the sequences are sampled. Thus, if in an index listing process sequence A were sampled as the first sequence, it would receive the index 1, but if it were sampled as the hundredth sequence, it would receive the index 100. This kind of indexing leads to incompatible indices for different index listing procedures and/or to incompatible indices derived from different data sets. In contrast, a hash function according to the present invention will always result in the same hash key for a sequence, irrespective of whether the sequence was sampled first, last or in the middle or whether the same sequence was sampled from different data sets.

A hash function according to the invention may e.g. be designed in such a manner that the probability of two sequences yielding the same hash key is less than 10⁻⁷, more preferably 10⁻⁹. Another possible criterion is that said probability of two hash functions yielding the same hash key is less than 1/M, more preferably less than 1/(1.000 M), where M is the number of sequences or data sets to be accommodated or an upper limit thereto.

Usually, a hash function employed in the invention will be a one-way hash function. Conventional algorithms that are known in the field of cryptography can be applied in the context of the present invention, especially the MD4 or the MD5 algorithm.

The typical length of sequences stored in sequence databases is about 100 to 1.000 elements for protein sequences and about 500 to 100.000 for nucleotide sequences. A typical order of magnitude of sequences that have to be accommodated in a non-redundant data source according to the invention is several millions.

The invention may provide that said hash function maps a sequence on a hash key having a length of at least 64 bit, preferably 128 bit.

Since 2⁶⁴ is of the order of magnitude of 10²⁰ and common one-way hash functions are devised in a manner that similar arguments yield vastly different function values, the likelihood that two sequences will result in the same hash key is negligibly small. This, of course, applies even more when choosing a 128 bit key. The length of the key should be less than 1 kbit, more preferably less than 500 bit, especially smaller than 200 bit.

Said collision-free hash function may have function values of a predetermined fixed number of bits. According to the preferred embodiment, it maps the sequence on to a data string of fixed length. The invention may provide that said reference data source is an index table, wherein each entry in said table comprises one hash key and one or more localization identifiers specifying the location of the data set of said basic data source comprising the sequence from which said hash key was generated.

The invention may especially provide that said index table is lexically sorted by the hash key in ascending or descending order.

The invention may provide that said information for retrieving information from said data set comprises or consists of information for retrieving or accessing said data set and/or information for accessing a certain location in said data set, e.g. a field of said data set.

The invention may provide that description information related to said data set and/or said sequence is stored in said reference data source together with said information for retrieving said data set comprising said sequence.

The invention may especially comprise the following steps:
- retrieving the biological sequence from a data set of one or more of said data sources and generating a hash key by applying a collision-free hash function on said biological sequence,
- if an entry exists in said non-redundant data source that is related to the hash key generated from said sequence, adding information for retrieving said data set to said entry,
- if the non-redundant database does not comprise an entry related to the hash key generated from said sequence, providing a new entry in said reference data source comprising said hash key and information for retrieving the data set.

The above-mentioned process can be done individually for each data set, i.e. one consecutively retrieves the biological sequence from the basic data set, checks the non-redundant data source as to whether there is a related entry and either adds retrieval information for said data set to an existing entry or generates a new entry, before handling the next data set.

The invention may, however, also provide that a plurality of data sets are processed in parallel. In this case, the step of providing a new entry in said reference data source may consist in providing a new entry comprising said hash key and information for retrieving all basic data sets comprising the sequence from which said hash key was generated, i.e. one single entry is created for a plurality of basic data sets comprising the related sequence.

The invention may provide that the steps of adding retrieval information for said data sets and providing a new entry or new entries comprise the step of creating a redundant version of the reference data source and removing the redundancies from said redundant version to yield the non-redundant reference data source by merging entries related to the same hash key.

The invention may provide the steps of
- for each of said data sets, creating an entry in a temporary data source comprising the hash key generated from the sequence contained in said data set and information for retrieving said data set, wherein said temporary data source comprises a plurality of entries, each of said entries comprising exactly one hash key,
- removing redundancies in said temporary data source such that each hash key is uniquely related to one single entry to yield said reference data source.

The invention may provide that said temporary data source is a data source that is created from the hash keys generated in the afore-mentioned manner and the corresponding information regarding the related data sets. This will be the normal course of proceeding, when a new non-redundant data source is to be created or if a small portion of a big non-redundant database is to be updated. The invention may, however, also provide that said temporary data source is created from an existing non-redundant data source by adding an entry for each data set for which the hash key relating to a sequence in said data set was generated.

In the above-mentioned procedure, the invention may provide that if two entries are found to relate to the same hash key, the information related to the hash key in a first of said two entries, especially information for retrieving said data set and possibly further description information, is included in the other entry and said first entry is deleted.

The invention may provide that a plurality of partial reference data sources are created from data sets of said one or more basic data sources, wherein each of said partial reference data sources consists of one or more entries, each of said entries comprising one single hash key, wherein each of said partial reference data sources relates to a group of basic data sets different from that to which the other partial reference data sources relate, wherein each of said partial reference data sources is free of redundancies such that any hash key contained in one of said partial reference data sources is a unique identifier for an entry in said partial reference data source
and in that said partial reference data sources are merged to one single unified reference data source in such a manner that said unified reference data source comprises the same information as said plurality of partial reference data sources and any hash key in said unified reference data source is a unique identifier for an entry in said unified reference data source.

In a specific embodiment each of said partial reference data sources is a table comprising one or more entries wherein a hash key is assigned to one or more identifiers of data sets comprising a sequence from which said hash key was derived, wherein said table is lexically sorted by the hash key in the same manner for all partial reference data sources (ascending or descending), and that said unified reference data source is created by a multiway merge algorithm.

According to one embodiment, the multiway merge algorithm proceeds in the way that the top or bottom position of each table is examined and that the top (or bottom) entry comprising the hash key that is lexically smallest (or largest) is removed to a unified reference table. If the top (or bottom) entries of two or more tables are the same, this means that there is a redundancy between the tables. In this case only one entry is created in the unified reference table and the further data comprised in said entries of said various partial reference tables, especially location identifiers and other information related to data sets, are merged into said one entry in said final table. The entry comprising the hash key that is the next smallest in the table from which an entry was removed is now the top (or bottom) element of the table and the process is repeated. This algorithm assures that the resulting table is lexically sorted.

For practical applications, when large databases are to be transferred into one single non-redundant database, one chooses about 50000 to 500000 basic data sets to be related to one temporary table. Especially, with a multiway merge algorithm as described above, remaining redundancies can be efficiently removed.

The invention may provide that a non-redundant unified database is created from said reference data source, wherein each entry in said non-redundant unified database is related to an entry of said reference data source by a 1:1 relation, wherein each entry in said non-redundant unified reference database contains at least the location identifiers of the data sets of the corresponding entry of the reference data source and wherein said non-redundant unified database does not comprise said hash keys as primary keys.

Especially primary keys of shorter length than the hash keys may be used in said non-redundant database.

A database in the sense of the invention can be any collection of data structured to comprise a plurality of entries, wherein each of said entries can be accessed by means of one or more entry identifiers, and which is structured in such a manner that the data in an entry can be retrieved by means of an entry identifier. A database in the sense of the invention can especially be a so-called flat file. Flat files are structured such that there are a plurality of entries identified by a certain syntax, wherein said entries can be individually accessed by means of a key. A database in the sense of the invention can, however, also be a structured collection of data comprising a plurality of fields, wherein each field contains one data element, e.g. value of a variable, and wherein fields are linked through structural relationships defined by a schema.

The invention may provide that at least some of the sequences corresponding to the hash keys are retrieved from a basic data source and added to the corresponding entries of said non-redundant database.

If a proper one way collision-free hash function is used, it is extremely unlikely that the same hash key will be assigned to two different sequences and that accordingly an entry in said unified database will comprise information related to different sequences. If, however, a function is used that is only weakly collision-free, i.e. there is a remaining realistic probability that the same value is assigned to two different sequences, the invention may provide a check on those entries in the non-redundant database relating to different basic data sets in order to verify that they actually relate to the same sequence. The same may apply if, for some reason, a high reliability of a non-redundant database is required.

In such cases, the invention may provide the step of retrieving those entries of the non-redundant unified database which comprise more than one location identifier, retrieving the sequence from the data set identified by each of said location identifiers contained in said entry and comparing the sequences thus retrieved. If accidentally two different sequences have been assigned to the same hash key and thus merged into the same entry, this will show up when the original sequences of the original data sets are compared. If a difference is detected, there will be suitably an alert of the database manager who will then split the entry and create separate entries related to different sequences in said non-redundant database. This could, of course, also be done automatically. One will suitably also alter the hash function to be used for subsequent updates, e.g. in the sense that different values are created for sequences having been found to result in the same hash key. Another way of checking errors is to include control information, e.g. the length of the sequence for each location identifier of a basic data set, and checking entries in the non-redundant database and/or in the reference data source as to whether this information is the same for all location identifiers in an entry.

According to a preferred embodiment the non-redundant unified database is in the FASTA format.

The invention may provide that said entries in said non-redundant unified database are ordered in the same order as the entries in said reference data source.

This order may be implied through by the sequence of entries or explicitly imposed on the entries of the non-redundant database, e.g. by an index in a field of an entry.

The invention may provide that said reference data source is maintained in a manner that it can be accessed by the same computer system as said unified non-redundant database.

The invention also provides a computer system comprising a reference data source and/or a non-redundant unified database obtainable by a method as previously described.

The invention furthermore provides a computer system comprising:
- software for accessing a database related to biological sequences, each entry in said database being uniquely related to one biological sequence,
- software for accessing a reference data source comprising a plurality of entries, each uniquely related to a biological sequence and having as unique identifier a hash key created by a collision-free hash function mapping said sequence on a data string of shorter length, wherein at least one of said entries comprises data derived from different basic data sets, wherein at least some of the entries in said database are uniquely related to at least some of the entries in said reference data source
- means for retrieving uniquely related entries in said reference data source and said non-redundant database.

The invention may provide that said computer system further comprises means for comparing related entries in said reference data source and said non-redundant database.

The invention may provide means for modifying said non-redundant database and/or said reference data source.

The invention may provide that said computer system comprises said non-redundant database and/or said reference data source.

The invention may, however, also provide that the reference data source or the database are remotely located from said computer system, but that said computer system is provided with means for remotely accessing said database and/or said reference data source.

Whereas in the preferred embodiments there is only one reference data source related to one database, one can, in principle, combine multiple reference data sources relating to different databases in one single reference data source, provided that entries related to different databases can be unambiguously identified. In such an embodiment, it may be that the same entry in a reference data source corresponds to related entries in different databases.

The invention may provide that each entry in said non-redundant database has a corresponding entry in said reference data source.

The computer system of the invention may also comprise
- means for creating a further reference data source, comprising a plurality of entries, each entry being uniquely related to a biological sequence and having as a unique identifier a hash key created by a collision-free hash function mapping said sequence on a data string of shorter length than the data representing said sequence, wherein each of said entries comprises a location identifier identifying a data set in a basic data source relating to the sequence identified by the hash key assigned to said entry.

The invention may provide means for modifying one or both of said reference data sources, if two entries related to the same hash key comprise different information and/or if one of said reference data sources comprises an entry related to a hash key that is not comprised in said other reference data source.

The invention may provide means for creating a data source comprising one or more entries, each of said entries relating to an entry in one of said two reference data sources that is missing from the other reference data source or comprises information not contained in a corresponding entry in said other reference data source.

The invention also provides a method of comparing a non-redundant data source, especially a non-redundant database, comprising a plurality of entries, each entry being uniquely related to a biological sequence, to data sets of one of more basic data sources related to said sequences, said method comprising
- providing a first reference data source comprising a plurality of entries, wherein each of said entries in said non-redundant database corresponds to exactly one entry in said reference data source, preferably also vice versa, each entry in said reference data source having as unique identifier a hash key obtainable by a collision-free hash function mapping the sequence to which said entry relates on a data string of shorter length, = = =
- providing a second reference data source comprising a plurality of entries, each of which is related to exactly one biological sequence and has as a unique identifier the hash key created by said hash function, when applied on the corresponding sequence, wherein each entry comprises information related to one or more data sets related to said sequence in one of said basic data sources,
- comparing said first and second reference data source as to whether entries in said two reference data sources relating to the same hash key comprise different information and/or as to whether an entry in said first or second reference data source is missing in the respective other reference data source.

According to this method, the non-redundant data source is compared with basic data sets by comparing corresponding reference data sources. It should be noted that the first reference data source could be the non-redundant data source itself.

The invention may provide that an entry in said first reference data source comprises at least partly the same data as the corresponding entry in said non-redundant data source. Said information related to one or more data sets may especially comprise information for retrieving information from a data set, e.g. the sequence contained therein, especially information for retrieving or accessing said data set or a certain location thereof.

The invention may provide that each entry in said second reference data source comprises a location identifier identifying the data set of the basic data source from which it was retrieved and each entry in said first reference data source comprises a location identifier identifying a data set in a basic data source comprising information regarding the sequence to which said entry is related and further comprising the step of comparing the location identifiers in entries in the two reference data sources identified by the same hash key.

The invention may provide that said non-redundant database is modified according to the result of said comparison.

Said step of modifying may comprise at least one of the following steps:
- if said second reference data source comprises an entry not contained in said first reference data source, creating an entry in said non-redundant database related to the sequence corresponding to said entry in the second reference data source not contained in the first reference data source, using information contained in said entry in said second reference data source,
- if said first reference data source comprises an entry not contained in said second reference data source, deleting the entry in said non-redundant database related to said entry in said first reference data source,
- if entries in said first and second reference data source related to the same hash key comprise different information, modifying the information contained in the related entry of said non-redundant database using the information contained in the related entry in said second reference data source.

The invention may provide the step of creating a comparison list listing
- entries in said first reference data source differing from entries related to the same hash key in said second reference data source, together with information indicating the difference and/or
- entries in said first reference data source not contained in said second reference data source and/or
- entries contained in said second reference data source not contained in said first reference data source, together with information about said entry in said second reference data source.

The invention may also provide the step of using said comparison list for modifying said non-redundant database.

The invention may provide that said non-redundant database comprises in each entry information indicating from which basic data source or part thereof the sequence information in said entry was derived.

The invention may comprise the steps of
- retrieving all data sets in a basic data source which is indicated in said non-redundant database to have provided sequence information,
- creating said second reference data source using all data sets relating to sequences in said basic data source and
- comparing the second reference data source thus created to the first reference data source.

In this way, an update of the non-redundant database is possible. Since the non-redundant data source should mirror all information in said basic data source, e.g. a database like SWISSPROT, a missing data set in the basic data source showing up in the comparison shows that a certain entry was removed from the basic data source and accordingly the related information should be removed from the non-redundant database. Likewise, if there is a new entry in said reference data source this means that a new sequence was entered and that the non-redundant database should be amended accordingly. Finally, if an entry in said second reference data source derived from said basic data source is different from the related entry in the first reference data source, this can mean that either a new data set relating to the same sequence was added to the basic data source and accordingly the information in the non-redundant database has to be enhanced by the additional information provided by said entry or that the corresponding data set in the basic data source was modified and that the non-redundant database has to be amended accordingly.

The invention may also provide that information from a new data source is added to the non-redundant database. In this case, one will, of course, not delete an entry in the non-redundant database that has no corresponding entry in the second reference data source. The same applies for cases where an update is made, but not from an updated version of the basic data source, but from a supplement to the basic data source. Big databases, like GENBANK or SWISSPROT, are updated only in intervals of three or four months. New information accumulating in the meantime is contained in a smaller database which forms a supplement to the original database. If an update is made from one of these supplementary databases, one will not delete an entry in the non-redundant database, if a corresponding entry does not show up in the reference data source derived from the supplementary database. If, however, the supplementary database indicates that some entries in the original database have been deleted, the invention may provide that the reference data source created from said supplementary database has a respective entry, identified by the related hash key and that an entry in said non-redundant database is to be deleted, if the second reference data source indicates that the underlying data set in the basic data source has been deleted.

Using the hash key according to the invention has a plurality of advantages. First, contrary to standard hashing techniques, the hash key, although relating to a shorter data string, is unique for all practical purposes. Second, one provides a unique identifier that can be used irrespectively of the structure and the keys of the underlying basic data sources and of the non-redundant database. Thus, information derived from vastly different data sources can be combined and compared, which is a significant advantage.

The method of the present invention consumes significantly less memory due to the improvements in detecting sequence identities and due to the overall process for assembling a new database version. In typical applications the maximum storage space needed is less than 1 GB. Likewise, execution speed is increased. If a redundant database comprising several millions of entries is transformed into a non-redundant database, typical calculation times are reduced by 20% to 40%. The present invention supports an incremental updating of non-redundant databases and requires a full scale recompilation only when a new non-redundant database is set up. Due to maintaining a reference data source comprising a digital fingerprint of the sequences, it is possible to provide incremental updates, both because of the reduced size of the data used for identifying a sequence and because a universal identifier is used that is applicable for all kinds of data sources related to sequences, irrespective of their structures, their keys or the like.

Subsequently the general outline of the algorithm according to one embodiment of the invention will be explained for the example that entries from one single source database are to be reconciled in one single non-redundant database. For the ease of explanation, it is assumed that each entry in said source database is represented in the so-called FASTA format. In the FASTA format the database is in ASCII text format. There is a header per sequence entry denoted by the first character of a line being the > sign. The end of the header is denoted by a carriage return line-feed. The remaining entries are sequence entries in normal ASCII format.

In a first step for each entry the related sequence is extracted from the source database. The a one-way hash function, e.g. the MD5 algorithm, is applied to each sequence, resulting in a hash key, subsequently also called "sequence fingerprint". A 64 bit hash algorithm should provide sufficient possibilities to accommodate all possible occurring sequences without having the same value of the hash function for two different sequences. Given the case, a 128 bit algorithm or an algorithm providing a hash key of even longer length may be used, as long as the hash key is still significantly shorter than the original data representing sequence.

As the next step the hash key calculated for the sequence is written to a temporary file together with the header of the related sequence entry in the FASTA format and a location identifier for identifying the entry in the source database. The hash key functions as an identifier for the entry in this file. Since the source database contained redundant entries, the hash key is not a unique identifier in said temporary file at this stage.

As a next step, redundant entries in the temporary file are merged into one single entry uniquely identified by a hash key. This may be conveniently done by selecting one of the redundant entries as a representative entry, adding, for all entries other than said representative entry, the location identifier for the entry in the source database, together with the related information retrieved from the FASTA header, to the existing identifier and descriptive information in said representative entry in such a manner that the location identifier and related description for different entries in the source database can be retrieved individually for each entry. In the most simple case one will simply put the location identifier and the related descriptive information behind the already existing data in the representative entry, separated by data indicating a new element, such as a blank. Conveniently, one will first sort the entries in the temporary file in lexical order according to this hash key and then combine subsequent entries having the same hash key.

The result of this procedure is a reference data source, subsequently also called "fingerprint index" which comprises in each entry the hash key or sequence fingerprint, the location identifiers, e.g. file offsets pointing to the sequence information in the source database, and the related header lines. The hash key is a unique identifier for entries in said reference data source. Conveniently, this fingerprint index is lexically sorted by the sequence fingerprint. With such sorting the fingerprint index can be compared and updated very efficiently.

If a large database is to be converted into a non-redundant database, it may be convenient to remove redundancies in a two step process. This process basically proceeds by processing only a number of sequences at the same time, storing the result in temporary files and treating each of said temporary files in the above-mentioned manner such that it contains only non-redundant entries. Subsequently, the plurality of temporary files is merged into one single file by a multiway merge algorithm, simultaneously removing the redundancies between the temporary files.

More specifically, the algorithm starts with reading a predetermined number N of entries, typically 50000 to 500000, from the source database, calculating the hash key for the sequence to which each entry relates and storing each of the hash keys thus generated in a temporary file together with a location identifier for the entry in the source database and the descriptive information retrieved from the FASTA header.

When said predetermined number N has been reached, the next N entries are written to a second temporary file, the next N entries into a third temporary file and so on. The entries to be written to one of said temporary files are lexically sorted according to the digital fingerprint (hash key) in the main memory and redundancies are removed, as described above. The remaining non-redundant entries are then written to the temporary file in this ordered sequence so that entries in said temporary files are lexically sorted according to the hash key and free of redundancies. At this stage one has a plurality of non-redundant temporary files.

The next step is to combine these temporary non-redundant files into one single non-redundant fingerprint index or reference data source. According to one embodiment, one uses a multiway merge algorithm, making use of the fact that each temporary file is lexically sorted according to its hash key. For the ease of explanation it is presumed that the lexically smallest hash key is on top and the lexically largest hash key is at the bottom and that processing starts at the top of the temporary files; although people skilled in the art will understand that the largest element could as well be at the top and/or processing could start from the bottom.

Assuming now that in each temporary file the entry comprising the lexically smallest hash key is at the top of each file (since each of the temporary files is free of redundancies between the entries, there is only one entry having the lexically smallest hash key), the top entries of all temporary files are examined as to which file comprises the lexically smallest hash key at its top position. If there is exactly one temporary file having the lexically smallest hash key at its top position, this entry is removed from the temporary file and put to the top position of a new file which will subsequently become the fingerprint index. If there are two or more temporary files having the same lexically smallest key at their top position, there is a redundancy between the data in these temporary files. In this case one selects the entry in one of the temporary files as a representative entry, adds the location identifier or location identifiers, together with related descriptive information derived from the FASTA header of the corresponding entries of the other temporary files to said representative entry, as described above, and puts this enhanced or amended entry at the top position of the file that is to become the fingerprint index. The entries related to said lexically smallest hash key are deleted from all temporary files that contain this hash key.

Whichever of the two possibilities occurred, at this stage one will have the entry or entries with the lexically smallest hash key removed from all temporary files and have one single related entry at the top position of the fingerprint index file.

This procedure is now repeated, i.e. one examines all entries now at the top of the temporary files as to which comprises the lexically smallest hash key, extracts the related entries or, in case of a redundancy, an entry merged from the related entries comprising the lexically smallest hash key and puts it at the next position in the fingerprint index table. This process is iterated until all entries in the temporary files have been transferred to the fingerprint index, whereby simultaneously all redundancies between the temporary files have been removed.

The fingerprint index one has now arrived at is essentially already some kind of non-redundant database, however with the flaw that the original sequence information is lost and, since the hash function is not or not easily invertible, cannot be retrieved other than by accessing a related entry in the source database. As this is frequently not desirable, one now creates a non-redundant database essentially by replacing the hash key by the full sequence information. Technically this can be done by setting up a new database, e.g. a flat file database in the FASTA format, wherein each entry comprises all information of the related entry of the fingerprint index, possibly together with some other, additional information, however without the hash key, by retrieving the sequence related to said entry from the source database on the basis of a location identifier contained in the respective entry in said fingerprint index of the fingerprint index and by adding this sequence to the entry of the non-redundant database. Thus, one will arrive at a non-redundant database, wherein each entry comprises a uniquely assigned sequence and which further comprises location identifiers comprising information for accessing entries related to said sequence in the source database plus descriptive information derived from the respective entries. Given the case, this information may be enhanced by further information added by a person maintaining the database or even by another automated process.

According to an important aspect of the invention the fingerprint index is permanently stored, e.g. together with the database. This provides a significant advantage when a non-redundant database has to be updated from an updated source database. For such update one calculates the fingerprint index for the updated source database, compares it to the fingerprint index of the old non-redundant database and implements the changes emerging from said comparison in the non-redundant database, simultaneously replacing the fingerprint index of the non-redundant database by the fingerprint index derived from the updated source database.

Implementing the differences of a supplementary database comprising updates to the source database can be effected e.g. by first implementing the changes implied by the supplementary database in the fingerprint index and then creating a new non-redundant database on the basis of the updated fingerprint index in the manner described before. Basically the same procedure is applied, if data from a new basic data source are added to the non-redundant database.

A different approach is to create a difference file indicating which entries have been modified, deleted or added. In one embodiment this difference file is implemented in the fingerprint index file by marking the related entries in the index files as modified or deleted and by adding new entries, marked as added. In this case, the index file would simultaneously comprise a record about the modifications made to the non-redundant database.

In another embodiment only the changes are recorded in a difference file which comprises information about the differences, together with an indicator where these differences are to be implemented. In case of an addition or an amendment of an existing entry, said indicator could be a unique identifier for entries in said non-redundant database. Especially if entries in said source database relate only to one sequence, the location identifier comprised in an entry of said non-redundant database is a unique identifier for said entry, since the database is non-redundant.

The invention may also provide that entries in the non-redundant database are assigned a unique identifier different from said location identifier and that said unique identifier is added to the related entry in said fingerprint index file and used to indicate the location where an amendment is to be made.

According to another embodiment the non-redundant database consists of sequential entries which are ordered in the same order as the entries in said fingerprint index file. In this case the position of a change can be established by indicating the number of the entry before which, after which or at which an entry has to be modified, deleted or added.

According to a further modification of the algorithm, the non-redundant database is divided into two or more parts which are each updated separately and eventually merged to result in the entire database. This is of advantage, if it is known in advance which parts of the non-redundant database are likely to be changed and which are less likely to be changed so that most of the update process happens with regard to one part of the database.

According to an embodiment of the invention, the fingerprint index file of all versions of the non-redundant database (or databases) are stored, which makes it possible to track the history of the database and especially to follow up amendments that were made over time by comparing the fingerprint index files.

The above-mentioned procedure is exemplified with reference to a simplistic example of eight nucleotide sequences. For the sake of simplicity, random successions of elements were chosen. Thus, these sequences do not have a specific biological function. In the example that follows it is presumed that the source database is written in the FASTA format with each entry identified by an identifier idₙ and a description descrₙ, where n is 1,2,3,...

It should be noted that realistic examples often comprise several millions of input sequences with a typical sequence length of several hundred characters up to some millions of characters (e.g. for complete genome entries in DNA sequence databases). Appropriate chunk sizes are then in the range of 50.000 up to 500.000.

For this example, the follwoing input data of the source database are provided in the FASTA format:

>id1 descr1
AGTCGATGCATGCTAGCTCAG
>id2 descr2
GATCGATATCGATCGGCATGCTAGCCCTGAAAAAAA
>id3 descr3
AGTCGATGCATGCTAGCTCAG
>id4 descr4
GTGACTG
>id5 descr5
AGTCGATGCATGCTAGCTCAG
>id6 descr6
ATCGATCGATAAGCTAGCTTTAGCTAGCTCGCATAGCTAG
CTAGCTACGCNNACTGACTAGCT
>id7 descr7
ATCGATCGATAAGCTAGCTTTAGCTAGCTCGCATAGCTAG
CTAGCTACGCNNACTGACTAGCT
>id8 descr8
CTATCGATCGATCGATCGATTCCGGGACTAAGCTA

In the next step, MD5 keys are calculated for each of the sequences shown above. The MD5 keys (base64-converted) for the eight sequences are:

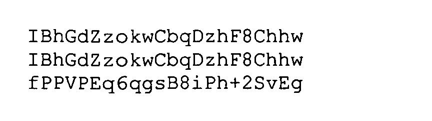

eeNJr3z2hOoYxiUeWibdbA
ago9+8cXdzKyWBIXnlaJAA
eeNJr3z2hOoYxiUeWibdbA
01iWHRWF9PDSQ0sO2Ls6+Q
eeNJr3z2hOoYxiUeWibdbA

As the next step, the eight sequences are divided in portions of four sequences, i.e. the entries id1 to id4 form a first portion (chunk) and the entries id5 to id8 form a second portion or chunk. In the next step two temporary files are created which comprise as columns the MD5 key of each sequence, calculated previously, the identifier for the respective entry (id1 to id8), the related description (descr1 to descr8), a file index (i.e. an index numbering FASTA files; for this example the index is always 1), the file offset (i.e. the position in the FASTA file forming the source database where the related entry can be found) and the sequence length. The columns are separated by tab stops. The entries in the temporary files are lexically sorted by the MD5 key in ascending order and redundancies are removed. Redundant entries are merged in that the entries in the columns of a redundant entry (other than the first column) are added as further columns to the remaining entry. In other words, the information of the other entry or entries (other than the hash key) is just appended to the information of the remaining entry. After this step there are two tables in two temporary files (chunk 1, chunk 2) which look as follows.

### Chunk 1:

01iWHRWF9PDSQ0sO2Ls6+Q   id4   descr4   1   129   8
ago9+8cXdzKyWBIXnlaJAA   id2   descr2   1   46   37
eeNJr3z2hOoYxiUeWibdbA   id1   descr1   1   12   22   id3   descr3   1   95   22

### Chunk 2:

IBhGdZzokwCbqDzhF8Chhw | id6 | descr6 | 1 | 183 | 65 | id7 | descr7 | 1 | 260 | 65
eeNJr3z2hOoYxiUeWibdbA | id5 | descr5 | 1 | 149 | 22
fPPVPEq6qgsB8iPh+2SvEg | id8 | descr8 | 1 | 337 | 36

In the next step the two chunks are merged into a single sorted file, i.e. the reference data source or "fingerprint index" of the input data. The merging algorithm for this example comprises the following steps:
1. Read the first entry from all chunk files
2. Find the entry or entries with the smallest MD5 key, merge entries having the same (smallest) key, and write the entry or merged entry into an output file
3. Read the next entry from the chunks from which the entries were taken in step 2.
4. Repeat at 2. if there are entries left to read.

The steps of merging the entries simultaneously implies the removal of redundancies. For example, it can be seen from the previous example the chunk 1 and chunk 2 comprise an entry related to the same sequence, identified by id1 in chunk 1 and id5 in chunk 2. For this example these entries will be at the top of both chunk 1 and chunk 2 in the fourth step of the merging algorithm. Since the MD5 keys are identical, there is a redundancy which means that id, description, file index, file offset and sequence length of the entry in the source database identified by id 5 are appended to the related entry in chunk 1, identified by id1, and written into the output file. For the above-mentioned example, the fingerprint index looks as follows.

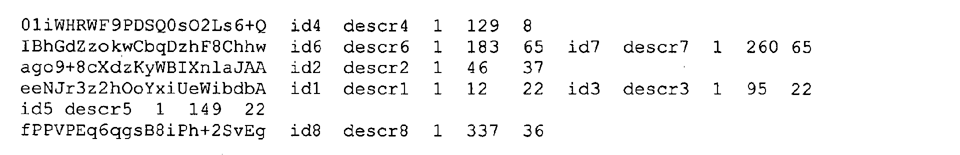

In order to build a final non-redundant database, for each entry in the fingerprint index file the sequence data are retrieved from the original entry in the source database, making use of the identifier, the file index and the file offset information contained in an entry. The non-redundant database is written in the FASTA format. The first identifier in an entry of the fingerprint index file becomes an identifier of the FASTA entry. This will be followed by the related description information in the header of the FASTA file, followed by the related id and description information for the other (redundant) entries in the source database.

If the above-mentioned algorithm is applied to the updated source database (including the above-mentioned amendments) the corresponding non-redundant database looks as follows:

>id4 descr4
GTGACTG
>id6 descr6 ; id7 descr7
ATCGATCGATAAGCTAGCTTTAGCTAGCTCGCATAGCTAG
CTAGCTACGCNNACTGACTAGCT
>id2 descr2
GATCGATATCGATCGGCATGCTAGCCCTGAAAAAAA
>id1 descr1 ; id3 descr3 ; id5 descr5
AGTCGATGCATGCTAGCTCAG
>id8 descr8
CTATCGATCGATCGATCGATTCCGGGACTAAGCTA

One should note that this database no longer contains the MD5 keys, but the full sequence. The resulting database contains only 5 entries (compared to 8 input entries). The IDs and descriptions of the redundant entries have been concatenated during the merging process.

The differences between two versions of a non-redundant database be very efficiently computed by comparing the sorted fingerprint indices. Let us assume that entry "id6" has been removed in the source database and two new entries "id9" and "id10" have been added to the source database, namely

>id9 descr9
ATGATTTTAGATTATAGATTATATAGGATAGGGATAGGAA
AAAA
>id10 descr10
GTGACTG

Note that "id9" is a new entry, whereas the sequence of "id10" is identical to "id4". The fingerprint index of the updated source database is

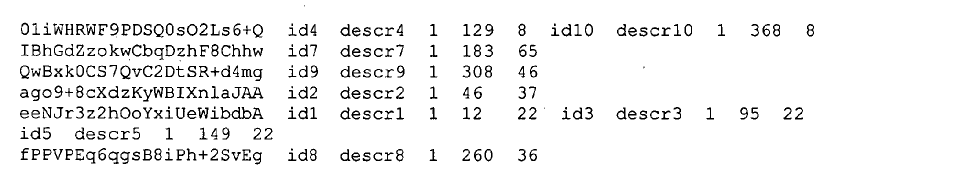

Since the fingerprint index of the previous version of the source database and thus of the existing non-redundant database was preserved, the old and the new fingerprint index can be compared in order to track the changes. Due to the ordering of the indices, this can be done by sequentially reading the entries from both fingerprint indices only once.
A diff file as follows is the result of this step:

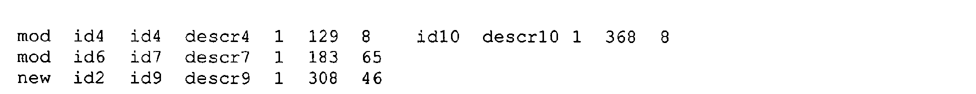

The first column specifies the kind of modification. Entries that disappear completely would be prefixed with del. The second column gives the primary ID of the entry in the old fingerprint index at which the modification has to take place. The following columns are formatted like the fingerprint index files. In the case of an addition, marked by "new" in the diff file, an addition is to be made immediately preceding the entry identified by the identifier in the second column, i.e. the new entry related to id9 has to be made prior to the entry related to id2.

Typically the differences between subsequent versions of databases are relatively small compared to the size of the complete database. This diff file can be used for two common scenarios:
1. Create a separate database which contains only the added non-redundant entries. In this example, the database would only contain entry "id9".
2. Construct the new non-redundant database by applying the diff file to the old non-redundant database.

The first scenario is easy to implement: all "Add' entries are extracted from the diff file, and the database is assembled as described in step c).

For the second scenario, the diff file is implemented as a table in main memory which has the sequence ids in the second column as keys. The old non-redundant database is now sequentially read. Whenever the identifier of an entry in the FASTA format is identical to the id in column 2 of the diff file, the respective modification is made. With the diff file as shown above, the entry identified by id6 is modified to consist only of id7 descr7, i.e. the previous data relating to id6 and descr6 are removed. Prior to the entry identified by id2 a new entry identified by id9 is added and at the entry identified by id4, new data are added so that the entire entry now reads id4 descr4; id10 descr10. One should note that already at this point the process will stop, because all entries in the diff file have been dealt with so that it is not necessary to go to the entry related to id1 and id8. It may also be possible to use a suitable search algorithm for each entry in the diff file for finding the related entries in the old non-redundant database. In a large database this may be more efficient than the previous sequential search approach.

The final, updated non-redundant database looks as follows:

>id4 descr4 ; id10 descr10
GTGACTG
>id7 descr7
ATCGATCGATAAGCTAGCTTTAGCTAGCTCGCATAGCTAG
CTAGCTACGCNNACTGACTAGCT
>id9 descr9
ATGATTTTAGATTATAGATTATATAGGATAGGGATAGGAA
AAAA
>id2 descr2
GATCGATATCGATCGGCATGCTAGCCCTGAAAAAAA
>id1 descr1 ; id3 descr3 ; id5 descr5
AGTCGATGCATGCTAGCTCAG
>id8 descr8

CTATCGATCGATCGATCGATTCCGGGACTAAGCTA

The result is the same as if a new non-redundant database had been created from the new fingerprint index file, as described previously. However, this procedure is several times faster than constructing the database in the manner described previously and should be used for update where changes are relatively small.

In order to improve the performance, one can also think of splitting the non-redundant database prior to updating, simultaneously creating partial fingerprint indices for each parts of the database, and updating the various parts independently of each other, followed by a final merge of the parts to the entire non-redundant database.

This step is optional but can improve the performance of building the fingerprint index file. If the non-redundant database is split into two (or more) parts, each part has its own fingerprint index. Before assembling the final non-redundant database by either using the "normal" method or the "updating" method, the indices of all parts are merged into a global fingerprint index file. This index is used to assemble the database or to compare it with previous versions.

This procedure pays off if it is known in advance that only one part (preferably the smallest one) has changed, since then only a small percentage of the MD5 keys have to be created. This is the case with a lot of biological databases, as they are divided into large "major" parts (which are seldomly updated) and smaller "new" parts, which are updated more frequently.

In order to determine the efficiency of the method according to the invention the above-mentioned procedure was applied to various biological databases. A non-redundant database was generated using the tool by NCBI ("NCBI tool") and using the method of the present invention ("fast NRDB"). The results were compared. The following non-redundant databases were examined.
1. *NR-PROT* (NR protein database), assembled form SWISSNEW, SWISSPROT, PDB, TREMBLNEW, TREMBL, PIR, GENPEPT and GENPEPTNEW databases.
2. *NR-EST* (NR EST nucleotide database), assembled from all ESTs in EMBLNEW, EMBL, GBNEW, GENEBANK databases.
3. *NR-GEN* (NR non-EST nucleotide database, genomic DNA), assembled from all non-EST sequences in EMBLNEW, EMBL, GBNEW, GENBANK.

Database contents were as of April 01, 2002. All computations were executed on a Unix server: COMPAQ ALPHA, OSF1 v5.1, using 1 CPU (400 MHz) and 4GB RAM. The following tables show execution time and memory consumption of fastNRDB compared to the NCBI nrdb tools.
• NR databases being computed from scratch.

| database | # input entries | # output entries | NCBI tool time [min] | fastNRDB time [min] | NCBI tool max mem [MB] | fastNRDB max mem [MB] |
|---|---|---|---|---|---|---|
| NR-PROT | 2.600.000 | 870.000 | 20 | 20 | 700 | 600 |
| NR-EST | 22.000.000 | 10.500.000 | 430 | 350 | 7000 | 600 |
| NR-GEN | 11.000.000 | 5.500.000 | 510 | 280 | 10000 | 600 |

• NR database (c.f. previous table) updated by sequences corresponding to a one week update volume.

| database | # input entries | # output entries | NCBI tool time [min] | fastNRDB time [min] | NCBI tool max mem [MB] | fastNRDB max mem [MB] |
|---|---|---|---|---|---|---|
| NR-PROT | 2.600.000 | 870.000 | - | 7 | - | 400 |
| NR-EST | 22.000.000 | 10.500.000 | - | 70 | - | 600 |
| NR-GEN | 11.000.000 | 5.500.000 | - | 70 | - | 600 |

The second table does not contain data relating to the NCBI tool, since an incremental update is not possible with this tool. If one were to compare the present invention with the NCBI tool with regard to a incremental update, one has to compare the time and memory space needed to do an incremental update with the time and memory space needed for the NCBI tool to establish an entirely new non-redundant database. One can see that the time for doing an incremental update according to the present invention is by a factor of 3 to 7 less than the time needed for implementing updates according to the prior art.

The features disclosed in the specification and the claims can be material for the realization of the invention in its various embodiments, both taken alone and in any combination thereof.

## Claims

1. Method for establishing or modifying a data source comprising a plurality of entries related to biological sequences that are non-redundant with regard to said sequences on the basis of a plurality of data sets of one or more basic data sources, each of said data sets comprising a biological sequence, said method comprising the steps of:
- retrieving for one or more data sets a biological sequence contained in the data set and generating a hash key from the biological sequence thus retrieved by applying a collision-free hash function, said hash function mapping the data representing said sequence onto a message of a length shorter than the length of the original data representing the sequence,
- for each of said data sets, adding information for retrieving information from said data set to an entry in a reference data source uniquely related to the hash key generated from said sequence contained in said data set, wherein a new entry in said reference data source is provided which comprises one unique hash key and information for retrieving the data set or data sets comprising the sequence from which said hash key was generated, if said reference data source does not comprise an entry related to said hash key,
such that each entry in said reference data source is uniquely identified by a hash key generated from a sequence.

2. Method according to claim 1, **characterized in that** said hash function maps a sequence on a hash key having a length of at least 64 bit.

3. Method according to claim 1 or 2, **characterized in that** description information related to said data set and/or said sequence is stored in said reference data source together with said information for retrieving said data set comprising said sequence.

4. Method according to claim 1 to 3, comprising the steps of
- for each of said data sets, creating an entry in a temporary data source comprising the hash key generated from the sequence contained in said data set and information for retrieving said data set, wherein said temporary data source comprises a plurality of entries, each of said entries comprising exactly one hash key,
- removing redundancies in said temporary data source such that each hash key is uniquely related to one single entry to yield said reference data source.

5. Method according to one of claims 1 to 4, **characterized in that** a plurality of partial reference data sources are created from data sets of said one or more basic data sources, wherein each of said partial reference data sources consists of one or more entries, each of said entries comprising one single hash key, wherein each of said partial reference data sources relates to a group of basic data sets different from that to which the other partial reference data sources relate, wherein each of said partial reference data sources is free of redundancies such that any hash key contained in one of said partial reference data sources is a unique identifier for an entry in said partial reference data source
and **in that** said partial reference data sources are merged to one single unified reference data source in such a manner that said unified reference data source comprises the same information as said plurality of partial reference data sources and any hash key in said unified reference data source is a unique identifier for an entry in said unified reference data source.

6. Method according to claim 5, **characterized in that** each of said partial reference data sources is a table comprising one or more entries wherein a hash key is assigned to one or more identifiers of data sets comprising a sequence from which said hash key was derived, wherein said table is lexically sorted by the hash key in the same manner for all partial reference data sources and that said unified reference data source is created by a multiway merge algorithm.

7. Method according to one of claims 1 to 6, **characterized in that** a non-redundant unified database is created from said reference data source, wherein each entry in said non-redundant unified database is related to an entry of said reference data source by a 1:1 relation, wherein each entry in said non-redundant unified reference database contains at least the location identifiers of the data sets of the corresponding entry of the reference data source and wherein said non-redundant unified database does not comprise said hash keys as primary keys.

8. Method according to claim 7, **characterized in that** at least some of the sequences corresponding to the hash keys are retrieved from a basic data source and added to the corresponding entries of said non-redundant database.

9. Method according to claim 7 or 8, **characterized in that** said entries in said non-redundant unified database are ordered in the same order as the entries in said reference data source.

10. Method according to one of claims 7 to 9, **characterized in that** said reference data source is maintained in a manner that it can be accessed by the same computer system as said unified non-redundant database.

11. Computer system comprising a reference data source obtainable by a method of one of claims 1 to 10 and/or a non-redundant unified database obtainable by a method according to one of claims 7 to 10.

12. Computer system comprising:
- software for accessing a database related to biological sequences, each entry in said database being uniquely related to one biological sequence,
- software for accessing a reference data source comprising a plurality of entries, each uniquely related to a biological sequence and having as unique identifier a hash key created by a collision-free hash function mapping said sequence on a data string of shorter length, wherein at least one of said entries comprises data derived from different basic data sets, wherein at least some of the entries in said database are uniquely related to at least some of the entries in said reference data source,
- means for retrieving uniquely related entries in said reference data source and said non-redundant database.

13. Computer system according to claim 12, **characterized by** means for modifying said non-redundant database and/or said reference data source.

14. Computer system according to one of claims 12 or 13, **characterized in that** said computer system comprises said non-redundant database and/or said reference data source.

15. Computer system according to one of claims 12 to 14, **characterized in that** each entry in said non-redundant database has a corresponding entry in said reference data source.

16. Computer system according to one of claims 12 to 15, **characterized by**
- means for creating a further reference data source, comprising a plurality of entries, each entry being uniquely related to a biological sequence and having as a unique identifier a hash key created by a collision-free hash function mapping said sequence on a data string of shorter length than the data representing said sequence, wherein each of said entries comprises a location identifier identifying a data set in a basic data source relating to the sequence identified by the hash key assigned to said entry.

17. Computer system according to claim 16, **characterized by** means for modifying one or both of said reference data sources, if two entries related to the same hash key comprise different information and/or if one of said reference data sources comprises an entry related to a hash key that is not comprised in said other reference data source.

18. Computer system according to one of claims 16 or 17, **characterized by** means for creating a data source comprising one or more entries, each of said entries relating to an entry in one of said two reference data sources that is missing from the other reference data source or comprises information not contained in a corresponding entry in said other reference data source.

19. Method of comparing a non-redundant data source comprising a plurality of entries, each entry being uniquely related to a biological sequence, to data sets of one of more basic data sources related to said sequences, said method comprising
- providing a first reference data source comprising a plurality of entries, wherein each of said entries in said non-redundant database corresponds to exactly one entry in said reference data source, each entry in said reference data source having as unique identifier a hash key obtainable by a collision-free hash function mapping the sequence to which said entry relates on a data string of shorter length,
- providing a second reference data source comprising a plurality of entries, each of which is related to exactly one biological sequence and has as a unique identifier the hash key created by said hash function, when applied on the corresponding sequence, wherein each entry comprises information related to one or more data sets related to said sequence in one of said basic data sources,
- comparing said first and second reference data source as to whether entries in said two reference data sources relating to the same hash key comprise different information and/or as to whether an entry in said first or second reference data source is missing in the respective other reference data source.

20. Method according to claim 19, **characterized in that** each entry in said second reference data source comprises a location identifier identifying the location of the data set of the basic data source from which it was retrieved and each entry in said first reference data source comprises a location identifier identifying the location of a data set in a basic data source comprising information regarding the sequence to which said entry is related and further comprising the step of comparing the location identifiers in entries in the two reference data sources identified by the same hash key.

21. Method according to claim 20, **characterized in that** said non-redundant database is modified according to the result of said comparison.

22. Method according to claim 21, **characterized in that** said step of modifying comprising at least one of the following steps:
- if said second reference data source comprises an entry not contained in said first reference data source, creating an entry in said non-redundant database related to the sequence corresponding to said entry in the second reference data source not contained in the first reference data source, using information contained in said entry in said second reference data source,
- if said first reference data source comprises an entry not contained in said second reference data source, deleting the entry in said non-redundant database related to said entry in said first reference data source,
- if entries in said first and second reference data source related to the same hash key comprise different information, modifying the information contained in the related entry of said non-redundant database using information contained in the related entry in said second reference data source.

23. Method according to one of claims 19 to 22, **characterized by** the step of creating a comparison list listing
- entries in said first reference data source differing from entries related to the same hash key in said second reference data source, together with information indicating the difference and/or
- entries in said first reference data source not contained in said second reference data source and/or
- entries contained in said second reference data source not contained in said first reference data source, together with information about said entry in said second reference data source.

24. Method according to claim 23, **characterized by** the step of using said comparison list for modifying said non-redundant database.

25. Method according to one of the claims 19 to 24, **characterized in that** an entry in said first reference data source comprises at least partly the same data as the corresponding entry in said non-redundant data source.
